# EUROPÄISCHE PATENTSCHRIFT

(11) **EP 3 135 206 B1**
(45) Veröffentlichungstag und Bekanntmachung des Hinweises auf die Patenterteilung: **15.01.2020**
(21) Anmeldenummer: 15183162.5
(22) Anmeldetag: 31.08.2015
(51) Int. Cl.: A61B 17/00, A61B 17/3207, A61M 31/00

(54) **VORRICHTUNG ZUR BEHANDLUNG DER VARIKOSE**
DEVICE FOR TREATING VARICOSE VEINS
DISPOSITIF DE TRAITEMENT DES VARICES

(43) Veröffentlichungstag der Anmeldung: 01.03.2017
(73) Patentinhaber: Gefässpraxis Dr. Erpen AG, 3930 Visp (CH)
(72) Erfinder: Erpen, Thomas, 3930 Visp (CH)
(74) Vertreter: AMMANN PATENTANWÄLTE AG BERN

(56) Entgegenhaltungen:
- EP-A1- 1 169 970
- EP-A1- 2 508 223
- EP-A2- 1 997 443
- WO-A1-2009/109967
- WO-A2-2004/112569
- WO-A2-2015/052703
- US-A1- 2003 120 256
- US-A1- 2011 218 494
- US-B1- 6 726 677

## Beschreibung

Die Erfindung betrifft eine Vorrichtung zur Behandlung der Varikose.

Die WO 2015/052703 A2 beschreibt eine Vorrichtung gemäss Oberbegriff des Anspruchs 1.

Die US 2011/0218494 A1 beschreibt ein Verfahren zur Abgabe eines therapeutischen und/oder diagnostischen Mittels an ein Gewebe durch Einfügen eines Katheters mit drei Ballonen in einen Körperhohlraum. Durch Inflatieren der beiden äusseren Ballone wird eine Kammer gebildet, in welche das Mittel eingeleitet wird, wobei dessen Druck erhöht wird durch Inflatieren des mittleren Ballons. Eine Anwendung bei der Behandlung der Varikose ist nicht beschrieben.

Neben chirurgischen Verfahren und der Sklerotherapie zur Behandlung der Varikose gewannen in den letzten Jahren endovenöse Verfahren (Laser, Radiofrequenz, ClariVein®) zunehmend an Bedeutung. Zur Ablation von Stammvenen entpuppen sich diese Verfahren als effizient und für den Patienten weniger belastend als die herkömmlichen operativen Verfahren. Sowohl Laser, Radiofrequenz als auch ClariVein® brauchen jedoch ein relativ teures Equipment. Neben den Kathetern sind Apparate zur Erzeugung des Lasers bzw. der Radiofrequenzwellen notwendig. Bei ClariVein® (vgl. z.B. WO 2013/090563 A1) wird durch einen sehr schnell rotierenden Draht mit einer speziell abgewinkelten Spitze innerhalb des Katheters, der gegen die Gefässinnenwand schlägt, ein Gefässspasmus erzeugt. Gleichzeitig wird flüssiges Verödungsmittel, Polidocanol, injiziert. Die sehr schnell rotierende Spitze führt ausserdem zu einem Verwirbeln des Verödungsmittels und damit zu einem gleichmässigen Kontakt mit der gesamten Veneninnenwand und verödet diese.

Insgesamt kommen bei den bekannten endovenösen Verfahren aufwändige Vorrichtungen zum Einsatz, welche die Behandlung teuer machen.

Es ist daher Aufgabe der vorliegenden Erfindung, eine Vorrichtung mit einfacherem Aufbau anzugeben, die eine wirksame Behandlung der Varikose ermöglicht.

Eine Vorrichtung, die diese Aufgabe löst, ist im Patentanspruch 1 angegeben. Die weiteren Patentansprüche geben bevorzugte Ausführungen an.

Die Vorrichtung gemäss Anspruch 1 umfasst einen Katheter, welcher einen Ballon mit mindestens einem Schneidelement ("Cutting Balloon") und welcher Seitenöffnungen aufweist. Über diese kann ein Sklerosierungsmittel in die Vene gelangen. Mittels des "Cutting Balloon" kann zumindest ein Teil der Veneninnenwand zerschnitten werden, um so die Zerstörung der Intima und Media der Vene herbeizuführen. Die Vorrichtung erlaubt somit eine Behandlung in Form einer endovenösen chemomechanischen Katheterablation. Aufgrund der Kombination einer Sklerotherapie mit einer mechanischen Zerstörung der insuffizienten Vene ist die Behandlungswirksamkeit gegenüber einer alleinigen Sklerotherapie heraufsetzbar. Im Vergleich zu den herkömmlichen endovenösen Verfahren ist kein zusätzlicher Einsatz von aufwändigen apparativen Hilfsmitteln notwendig. Insgesamt ist bei der erfindungsgemässen Vorrichtung der Aufbau relativ einfach und der apparative Aufwand reduzierbar, wobei eine wirksame sowie zeit- und kostengünstige Behandlung ermöglicht wird.

Weitere spezifische Konstruktionsmerkmale der Vorrichtung und deren Vorteile sind aus folgender Beschreibung und Zeichnungen von Ausführungsbeispielen ersichtlich. Es zeigen
Fig. 1 ein erstes Ausführungsbeispiel eines Aussenkatheters einer Vorrichtung zur Behandlung der Varikose in einer teilweise geschnittenen Seitenansicht,
Fig. 2 eine Seitenansicht eines Innenkatheters, der im Aussenkatheter gemäss Fig. 1 aufnehmbar ist,
Fig. 3 den proximalen Endabschnitt des Aussenkatheters gemäss Fig. 1 in einer Ansicht von oben,
Fig. 4 den proximalen Endabschnitt gemäss Fig. 3 in einem Längsschnitt, so dass ein Schneidelement von der Seite zu sehen ist,
Fig. 5 den proximalen Endabschnitt des Aussenkatheters gemäss Fig. 1 in einer teilweise geschnittenen Seitenansicht,
Fig. 6 ein zweites Ausführungsbeispiel eines Aussenkatheters einer Vorrichtung in einer teilweise geschnittenen Seitenansicht,
Fig. 7 einen Innenkatheter in einer teilweise geschnittenen Seitenansicht, der im Aussenkatheter gemäss Fig. 6 aufnehmbar ist,
Fig. 8 schematisch ein Bein mit der Stammvene, in welche die Katheter aus Fig. 1 und 2 eingefügt sind,
Fig. 9 das distale Ende der zusammengefügten Katheter aus Fig. 1 und 2 mit angeschlossenen Spritzen, und
Fig. 10 das proximale Ende der zusammengefügten Katheter aus Fig. 1 und 2, wobei der Aussenkatheter geschnitten dargestellt ist.

### Erstes Ausführungsbeispiel

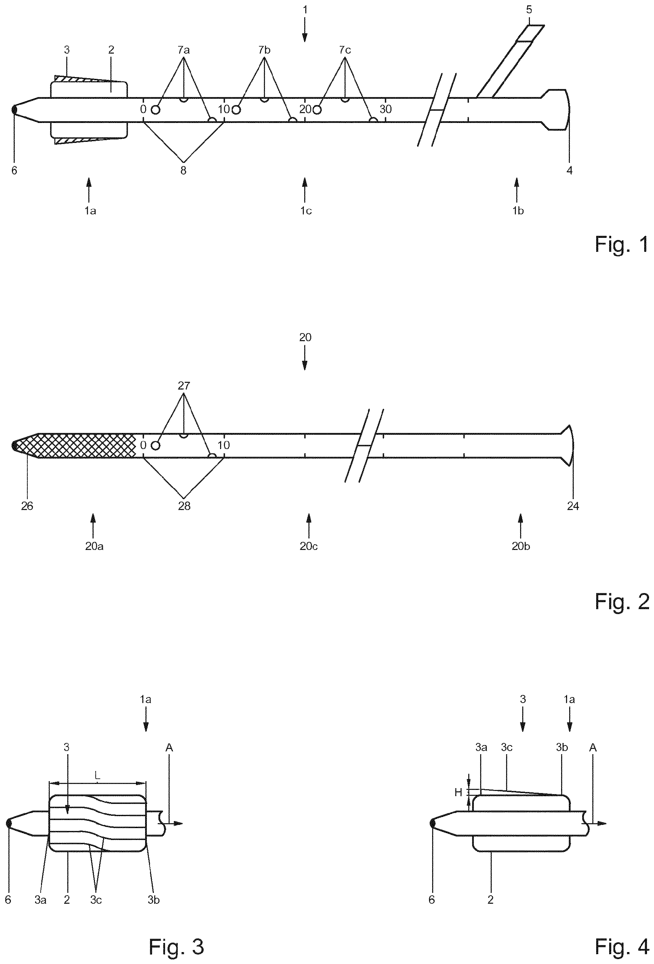

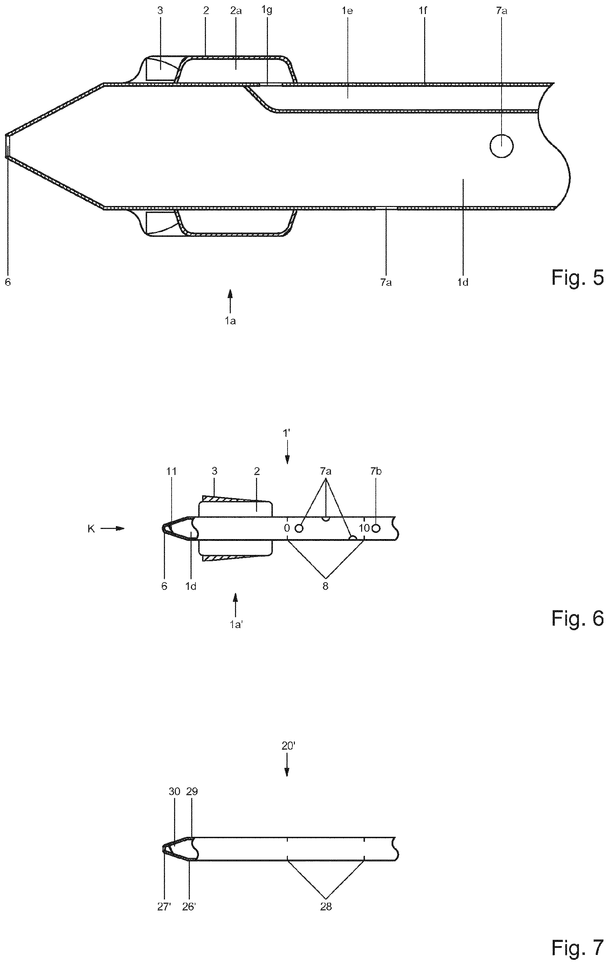

Fig. 1 zeigt einen ersten Katheter 1 (im Folgenden auch "Aussenkatheter"), der als Vorrichtung zur endovenösen Behandlung der Varikose dient und der in eine Vene einführbar ist. Der Katheter 1 weist einen proximalen Endabschnitt 1a mit einem Ballon 2 auf, welcher in der Fig. 1 in einer Schnittansicht und im expandierten Zustand gezeigt ist und welcher Schneidelemente 3 trägt, und einen distalen Endabschnitt 1b mit einer Eingangsöffnung 4 und einem Anschluss 5, welcher über einen Nebenkanal 1e ("Lumen") fluidisch mit dem Ballon 2 verbunden ist (vgl. Fig. 5). Am Anschluss 5 ist z.B. eine Spritze oder drgl. anschliessbar, um über den Nebenkanal 1e ein Fluid in den Ballon 2 zu pumpen bzw. aus diesem wieder abzulassen und so ein In- und Deflatieren des Ballons 2 zu ermöglichen. Um einen Patienten durch eine etwaige Undichtigkeit des in die Vene eingeführten Ballons 2 nicht zu gefährden, wird nicht Luft als Fluid zum Inflatieren verwendet, sondern eine Flüssigkeit, beispielsweise eine Natriumchloridlösung. Der proximale Endabschnitt 1a umfasst ein sich verjüngendes Ende, welches mit einer durchgehenden Endöffnung 6 versehen ist. Diese ist über einen Hauptkanal 1d ("Lumen") fluidisch mit der Eingangsöffnung 4 verbunden (vgl. Fig. 5).

Der Katheter 1 ist fenestriert. Zu diesem Zweck weist er zwischen den beiden Endabschnitten 1a und 1b einen Zwischenteil 1c auf, der mit Seitenöffnungen 7a-7c ("Katheterfenster") versehen ist. Diese sind um den Umfang des Katheters 1 herum verteilt angeordnet und über den Hauptkanal 1d mit der Eingangsöffnung 4 fluidisch verbunden. Bei der Anwendung ist über die Seitenöffnungen 7a-7c ein Sklerosierungsmittel in die zu behandelnde Vene injizierbar. Zwei benachbarte Seitenöffnungen 7a-7c sind axial und radial versetzt zueinander angeordnet. Der Versatz erlaubt eine möglichst homogene Verteilung von Sklerosierungsmittel in der Vene.

Entlang des Zwischenteils 1c sind auf dem Katheterschaft Markierungen 8 vorgesehen, die beispielsweise in regelmässigen Abständen angebracht sind und u.a. Angaben darüber liefern, wie weit der Katheter 1 in eine Vene eingeführt ist. In der Variante gemäss Fig. 1 sind Markierungen 8 in Form von Strichen und Zahlen 0, 10, 20, 30 zu sehen. Natürlich sind auch andere Arten von Markierungen möglich.

Die Seitenöffnungen 7a-7c sind in Gruppen angeordnet, so dass der Zwischenteil 1c in Abschnitte unterteilt ist, die jeweils die gleiche Anordnung an Seitenöffnungen 7a-7c aufweist. In der Variante gemäss Fig. 1 ist z.B. zwischen dem Abschnitt 0 bis 10 eine Gruppe mit drei Seitenöffnungen 7a zu sehen. Dieselbe Anordnung an Seitenöffnungen wiederholt sich im Abschnitt 10 bis 20 bzw. 20 bis 30. Die Seitenöffnungen 7a, 7b, 7c in der jeweiligen Gruppe sind radial um einen Winkel versetzt angeordnet. Beim Beispiel mit drei Seitenöffnungen kann dieser Winkel 120 Grad betragen. Es ist jedoch auch eine ungleichmässige radiale Verteilung denkbar. Im Weiteren kann die Anzahl der Seitenöffnungen 7a-7c pro Gruppe bzw. Abschnitt anders als in Fig. 1 gezeigt sein und eins, zwei oder mehr betragen. Da die Seitenöffnungen 7a-7c durch die Aussenwandung des Katheters 1 hindurchreichen, sind Anzahl und Anordnung so gewählt, dass genügend Platz bleibt, um zumindest einen abgeschlossenen Nebenkanal vom Anschluss 5 zum Ballon 2 vorsehen zu können (vgl. Nebenkanal 1e in Fig. 5).

Über die Eingangsöffnung 4 des Aussenkatheters 1 ist ein zweiter Katheter 20 (im Folgenden auch "Innenkatheter") einführbar, wie er in Fig. 2 gezeigt ist. Der Katheter 20 weist einen proximalen Endabschnitt 20a und einen distalen Endabschnitt 20b mit einem Anschluss 24 auf. Der proximale Endabschnitt 20a ist durch eine geschlossene Wandung gebildet. Insbesondere ist im Gegensatz zum Aussenkatheter 1 das sich verjüngende Ende 26 frei von einer Endöffnung. Das geschlossene Ende 26 des Innenkatheters 20 erlaubt ein Verschliessen des Lumen an der Spitze des Aussenkatheters 1, wodurch bei der Anwendung von Sklerosierungsmittel ein Wegfliessen nach proximal verhindert wird.

Zwischen den beiden Endabschnitten 20a und 20b weist der Katheter 20 einen Zwischenteil 20c auf, der einen Innenkanal ("Lumen") aufweist und der mit Seitenöffnungen 27 versehen ist. Diese sind über den Innenkanal fluidisch mit dem Anschluss 24 verbunden. Wie aus Fig. 2 ersichtlich, ist der Katheter 20 anschliessend zum proximalen Endabschnitt 20a lediglich auf einem Teilabschnitt mit Seitenöffnungen 27 versehen, während der übrige Katheterschaft keine Öffnungen aufweist. Es ist somit im Gegensatz zum Aussenkatheter 1 nur eine Gruppe an Seitenöffnungen 27 vorgesehen. Vorzugsweise entspricht die Anzahl und/oder Anordnung der Seitenöffnungen 27 der Anzahl bzw. Anordnung der ersten Gruppe an Seitenöffnungen 7a beim Aussenkatheter 1. Im Beispiel gemäss Fig. 2 sind drei Seitenöffnungen 27 zu sehen, die radial und axial versetzt zueinander angeordnet sind ähnlich wie die Seitenöffnungen 7a in Fig. 1. Je nach Auslegung kann die Anzahl der Seitenöffnungen 27 eins, zwei oder mehr betragen.

Entlang des Zwischenteils 20c des Katheters 20 sind Markierungen 28 vorgesehen, die beispielsweise in regelmässigen Abständen angebracht sind und u.a. Angaben darüber liefern, wie weit der Katheter 20 in den Aussenkatheter 1 eingeführt ist. Als Markierungen 28 dienen z.B. Striche, Zahlen, etc.

Bei der Anwendung ist der Innenkatheter 20 im Aussenkatheter 1 aufgenommen und wird dann abschnittsweise zurückgezogen. Dabei befinden sich die Seitenöffnungen 28 zuerst in der Nähe der Seitenöffnungen 7a, anschliessend bei den Seitenöffnungen 7b, usw. Der verschlossene Endabschnitt 20a des Innenkatheters 20 dichtet dabei den Hauptkanal 1d des Aussenkatheters 1 zwischen den Enden 6 und 26 ab. Über den Anschluss 24 und die Seitenöffnungen 27 und 7a-7c ist somit ein Sklerosierungsmittel abschnittsweise in die zu behandelnde Vene einleitbar.

Fig. 3 und 4 zeigen die Ausgestaltung des proximalen Endabschnitts 1a des Aussenkatheters 1 im Detail, wobei der Ballon 2 im expandierten Zustand dargestellt ist. Im Folgenden wird als "axial" die Richtung bezeichnet, in welcher die Achse A verläuft, entlang welcher sich der Aussenkatheter 1 vom Endabschnitt 1a zum Endabschnitt 1b erstreckt, während "radial" quer zur Achse A ist.

Der Ballon 2 ist ein "Cutting Balloon" und weist zu diesem Zweck ein oder mehrere Schneidelemente 3 ("Klingen") auf. In Fig. 3 sind mehrere Schneidelemente 3 gezeigt, die sich parallel zueinander in axialer Richtung erstrecken und die um den Ballon 2 herum angeordnet sind. Wie ersichtlich, verläuft ein jeweiliges Schneidelement 3 in axialer Richtung A gesehen nicht gerade, sondern seine Schnittkante 3c weist zumindest entlang eines Abschnitts einen gekrümmten Verlauf auf.

Es sind vielfältige Formen an Verläufen denkbar. Beispielsweise kann die Schnittkante 3c so gekrümmt sein, dass sie sich um die Achse A windet, z.B. schraubenförmig. Es ist auch denkbar, dass in Richtung der Achse A gesehen die Schnittkante 2a einen Abschnitt mit einem geraden axialen Verlauf aufweist, der über einen gekrümmten Zwischenabschnitt wieder in einen geraden Abschnitt übergeht. Es ist auch denkbar, nur ein einzelnes Schneidelement 3 vorzusehen, welches um die Achse A verläuft.

Der ungerade Verlauf eines Schneidelements 3 führt dazu, dass gesehen in der axialen Richtung A die Enden 3a und 3b eines Schneidelements 3 um einen Winkel radial versetzt angeordnet sind, der grösser als 0 Grad ist. Bevorzugt beträgt der Winkel mindestens 10 Grad und besonders bevorzugt mindestens 20 Grad. Im Weiteren kann der Verlauf so sein, dass besagter Winkel weniger als 360 Grad ist. Bevorzugt beträgt er höchstens 180 Grad und besonders bevorzugt höchstens 90 Grad.

Wie aus Fig. 4 ersichtlich, verändert sich die Form eines Schneidelements 3 auch quer zur Achse A, indem die Schneidkante 3c auf einer Höhe verläuft, die in Richtung zum distalen Endabschnitt 1b hin abnimmt. Das distale Ende 3b des Schneidelements 3 befindet sich somit näher bei der Mitte der Achse A als das proximale Ende 3a. Im vorliegenden Ausführungsbeispiel ist das Schneidelement 3 keilförmig ausgebildet. Die maximale Höhe H eines Schneidelements 3 ist typischerweise im Bereich von 0.5 - 1.5 mm.

Der Ballon 2 erstreckt sich axial auf einer Länge L, welche typischerweise im Bereich von 5 - 30 mm liegt.

Die sich axial bzw. radial veränderliche Formgebung eines Schneidelements 3 erlaubt eine umfassende mechanische Einwirkung auf eine Veneninnenwand, wenn der in die Vene eingeführte Katheter 1 wieder herausgezogen wird. Dabei graben sich die sich verjüngenden Schneidkanten 3c ähnlich eines Pflugs allmählich in die Veneninnenwand ein. Es wird daher eine abrupte mechanische Einwirkung vermieden, so dass eine schmerzfreiere Behandlung möglich ist, die unter Umständen auch ohne örtliche Betäubung in Form einer Tumeszenzanästhesie erfolgen kann. Dies ist z.B. dann der Fall, wenn Aethoxysklerol® als Sklerosierungsmittel eingesetzt wird, das ja auch ein Lokalanästhetikum ist.

Fig. 5 zeigt den proximalen Endabschnitts 1a des Aussenkatheters 1 in einem teilweise geschnittenen Längsschnitt, wobei der Ballon 2 im unaufgeblähten Zustand dargestellt ist. Der Katheter 1 umfasst eine Aussenwandung 1f, die den Hauptkanal 1d definiert. In diesen ist bei der Behandlung der Innenkatheter 20 aufnehmbar. Bei Bedarf kommt vorgängig ein Draht als Führungshilfe zum Einsatz, der in die Vene eingeführt wird und über welchen der Aussenkatheter 1 geschoben wird, so dass der Draht durch die Endöffnung 6, den Hauptkanal 1d und die Eingangsöffnung 4 hindurch verläuft.

Der Ballon 2 ist durch eine Hülle gebildet, welche um die Aussenwandung 1f herum verläuft und welche eine Kammer 2a umgibt. Diese ist über mindestens eine in der Aussenwandung 1f gebildete Durchgangsöffnung 1g mit dem Nebenkanal 1e verbunden, der zur Eingangsöffnung 4 führt. An der Hülle 2 sind die Schneidelemente 3 befestigt. Die Hülle des Ballons 2 ist im unaufgeblähten Zustand so zusammengefaltet, dass sich in radialer Richtung eine möglichst geringe Ausdehnung ergibt.

### Zweites Ausführungsbeispiel

Die Vorrichtung mit den Kathetern 1 und 20 gemäss Fig. 1 und 2 ist besonders geeignet zur Behandlung von grosskalibrigen Venen, z.B. Stammvenen. Fig. 6 und 7 zeigen ein anderes Ausführungsbeispiel der Vorrichtung mit Kathetern 1', 20', welche besonders bei kleineren Venen, z.B. Seitenästen, einsetzbar ist.

Fig. 6 zeigt einen Teil des Aussenkatheters 1', der dieselben Elemente 2-8 aufweist wie der Aussenkatheter 1 gemäss Fig. 1. Im Unterschied zu diesem ist beim proximalen Endabschnitt 1a' im Hauptkanal 1d zusätzlich ein Einwegventil 11 z.B. in Form einer Rückschlagklappe angeordnet. Das Einwegventil 11 dient zum Verschliessen der Endöffnung 6, so dass diese in der kaudalen Richtung, d.h. von aussen in den Hauptkanal 1d hinein (vgl. Pfeil K in Fig. 6) passierbar ist, jedoch nicht in der Gegenrichtung. Somit ist z.B. ein Draht, der als Führungshilfe in eine zu behandelnde Vene eingeführt worden ist, in der Richtung K über die Eingangsöffnung 6 im Hauptkanal 1d des Aussenkatheters 1' aufnehmbar, währenddessen Sklerosierungsmittel, welches sich im Hauptkanal 1d befindet, nicht über die Endöffnung 6 nach aussen gelangen kann.

Fig. 7 zeigt den Innenkatheter 20', wobei der distale Endabschnitt 20b mit dem Anschluss 24 jenem des Innenkatheters 20 gemäss Fig. 2 entspricht. Im Unterschied zu diesem ist der Innenkatheter 20' frei von Seitenöffnungen 27. Somit ist die Aussenwandung 29, welche den zum Anschluss 24 führenden Kanal 30 umgibt, geschlossen ausgebildet. Das proximale Ende 26' des Innenkatheters 20' mündet in einer durchgehenden Austrittsöffnung 27'.

Ist der Innenkatheter 20' im Aussenkatheter 1' aufgenommen, so ist Sklerosierungsmittel über den Anschluss 24 in den Hauptkanal 30 einleitbar, von wo aus es über die Austrittsöffnung 27' und über die Seitenöffnungen 7a-7c des Aussenkatheters 1' in die zu behandelnde Vene gelangen kann. So wie beim ersten Ausführungsbeispiel ist eine Vene abschnittsweise behandelbar, indem abwechselnd Sklerosierungsmittel injiziert und der Innenkatheter 20' ein Stück weit zurückgezogen wird. Dabei kommt nacheinander die Austrittsöffnung 27' in der Nähe der Seitenöffnungen 7a, 7b, 7c, etc. zu liegen.

Zur Fertigung der Katheter 1, 1, 20, 20' sind gängige Materialien einsetzbar, die sterilisierbar sind. Die Wandungen, welche die Kanäle der Katheter 1, 1, 20, 20' definieren, sind z.B. aus Kunststoff fertigbar, so dass ein flexibler Schlauch ausbildbar ist. Vorzugsweise sind die Katheter 1, 20 bzw. 1,' 20' für einen Einweggebrauch ausgelegt und werden in steriler Form in Verpackungen bereitgestellt.

### Anwendungen:

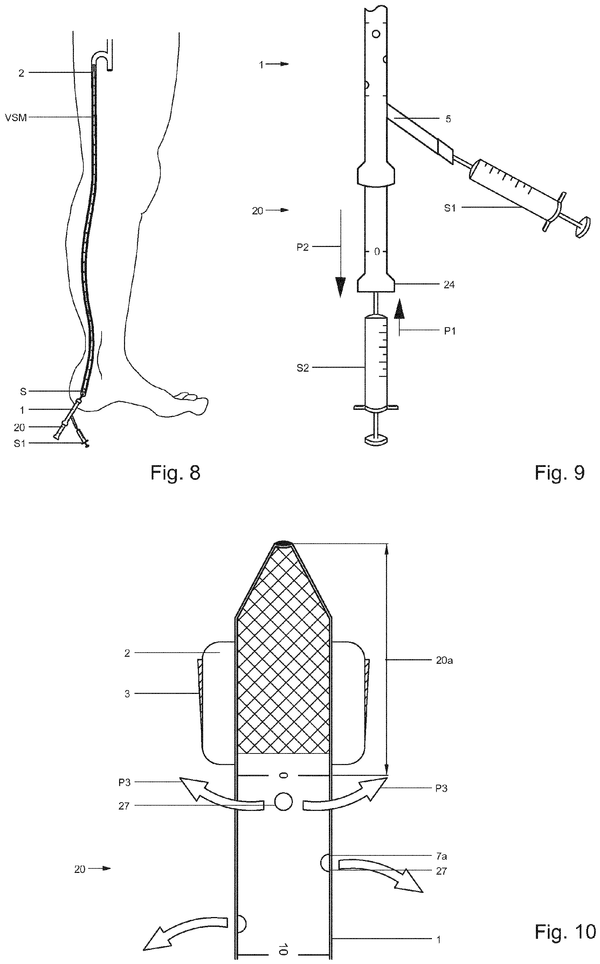

Nachfolgend wird anhand der Fig. 8-10 eine mögliche Anwendung der Vorrichtung mit den Kathetern 1 und 20 genauer erläutert.

Fig. 8 zeigt ein Bein mit der Vena saphena magna VSM und einer Schleuse S, die mittels Sedlinger-Technik in die Vene eingeführt wurde.

### 1. Schritt

Der Katheter 1 wird über die Schleuse S eingeführt, ultraschall-kontrolliert über die Stammvene VSM vorgeschoben und die Spitze 1.5 cm kaudal der Crosse platziert. Anschliessend wird mittels einer am Anschluss 5 angebrachten Spritze S1 der Ballon 2 inflatiert. Er bleibt dies während der gesamten Sklerosierungsphase (2. Schritt).

Ist ein direktes Vorschieben des Katheters 1 erschwert, kann vorgängig ein (in den Fig. 8-10 nicht dargestellter) Draht als Führungshilfe in die Vene VSM eingeführt und anschliessend der Katheter 1 über diesen eingeschoben werden.

Eine etwaige Dissektion beim Anbringen des Katheters 1 und/oder des Drahtes ist unproblematisch, da ja die Vene sowieso zerstört werden soll.

Nach Setzen des Aussenkatheters 1 wird der Draht, sofern vorhanden, entfernt und der Innenkatheter 20 in den Hauptkanal 1d eingeführt. Wird kein Draht verwendet, so kann der Innenkatheter 20 bereits zu Beginn im Aussenkatheter 1 eingesetzt sein, so dass beide Katheter 1, 20 zusammen in die Vene einführbar sind.

### 2. Schritt

Es folgt die Sklerosierung. Mittels einer am Anschluss 24 angebrachten Spritze S2 wird über den Innenkatheter 20 das Sklerosierungsmittel (vorzugsweise als Schaum, z.B. Aethoxysklerol® 1% oder ein anderes Polidocanol enthaltendes Mittel) zugeführt. Der Innenkatheter 20 wird in 10 cm-Schritten herausgezogen. Pro 10 cm wird jeweils 1 ml Aethoxysklerol® 1% als Schaum appliziert und ca. 1 Minute gewartet. Es wird so weiterverfahren, bis der gesamte Innenkatheter 20 entfernt ist.

In Fig. 9 ist das Applizieren durch den Pfeil P1 und das Zurückziehen des Innenkatheters 20 durch den Pfeil P2 angedeutet.

In Fig. 10, in welcher der Aussenkatheter 1 geschnitten dargestellt und der Innenkatheter 20 ganz darin eingeführt ist, sind durch die Pfeile P3 der Austritt des Sklerosierungsmittels aus den Seitenöffnungen 27 sowie 7a angedeutet. Wie weiter oben erläutert, ist das Lumen des Innenkatheters 20 am proximalen Ende verschlossen ausgebildet. Dieser verschlossene Bereich, der z.B. bis zur 0-Marke geht, ist in Fig. 10 mit dem Doppelpfeil bei 20a angedeutet

### 3. Schritt

Die Einwirkung des Sklerosierungsmittels hat zu einem Vasospasmus geführt. Zudem sollte auch die Schmerzempfindlichkeit vermindert sein. Der Ballon 2 wird für ca. 20 Sekunden deflatiert und anschliessend wieder inflatiert. Die Vene VSM kann sich so "entleeren". Nun wird der Katheter 1 mit dem inflatierten Ballon 2 langsam zurückgezogen (ca. 3 Sekunden für 10 cm). Beim Zurückziehen sollte der Widerstand gut spürbar sein, ein "Herausreissen" sollte aber vermieden werden. Durch die mechanische Einwirkung eines Schneidelements 3 auf dem Ballon 2 wird die Intima und Media der Vene zerstört. Die spezielle Formgebung eines Schneidelements 3 erlaubt eine wirksame Zerstörung der Veneninnenwand, die beim Zurückziehen des Ballons 2 in multiple Fragmente zerschnitten wird.

Die Vorrichtung gemäss dem zweiten Ausführungsbeispiel ist analog einsetzbar wie oben beschrieben. Hier kann auch bei Verwendung eines Drahtes der Innenkatheter 20' bereits zu Beginn im Aussenkatheter 1' eingesetzt sein, da beim Einführen der Katheter 1', 20' in eine Vene der Draht durch die Endöffnungen 6, 27' und das Einwegventil 11 in das Innere das Katheters 20' gelangen kann.

Aus der vorangehenden Beschreibung sind dem Fachmann zahlreiche Abwandlungen zugänglich, ohne den Schutzbereich der Erfindung zu verlassen, der durch die Ansprüche definiert ist.

In einer einfacheren Ausführungsform ist es z.B. denkbar, den Innenkatheter 20, 20' wegzulassen und nur einen Katheter mit einem "Cutting Balloon" 2, 3 und den Seitenöffnungen 7a-7c vorzusehen.

## Patentansprüche

1. Vorrichtung zur Behandlung der Varikose, umfassend einen Katheter (1, 1'), welcher in eine zu behandelnde Vene einführbar ist, wobei der Katheter Seitenöffnungen (7a-7c) aufweist, durch welche hindurch ein Sklerosierungsmittel in die Vene einleitbar ist, **dadurch gekennzeichnet, dass** am proximalen Ende (1a, 1a') des Katheters ein Ballon (2) mit mindestens einem Schneidelement (3) zum Zerschneiden zumindest eines Teils der Veneninnenwand angeordnet ist.

2. Vorrichtung nach Anspruch 1, wobei sich der Katheter (1, 1') in der axialen Richtung (A) erstreckt und wobei das mindestens eine Schneidelement (3) eine Schneidkante (3c) aufweist, die zumindest im inflatierten Zustand des Ballons (2) einen Verlauf aufweist, der sich in axialer und/oder radialer Richtung verändert.

3. Vorrichtung nach einem der vorangehenden Ansprüche, wobei das Schneidelement (3) zumindest auf einem Teilabschnitt gekrümmt ist und/oder eine Höhe aufweist, die zum distalen Ende (1b) hin gesehen abnimmt.

4. Vorrichtung nach einem der vorangehenden Ansprüche, wobei gesehen in der axialen Richtung (A), in welcher sich der Katheter (1, 1') erstreckt, die Enden (3a, 3b) des Schneidelements (3) um einen Winkel radial versetzt angeordnet sind, der grösser als 0 Grad und/oder kleiner als 360 Grad ist, bevorzugt beträgt der Winkel mindestens 10 Grad und/oder höchstens 180 Grad, besonders bevorzugt beträgt der Winkel mindestens 20 Grad und/oder höchstens 90 Grad.

5. Vorrichtung nach einem der vorangehenden Ansprüche, wobei zwei benachbarte Seitenöffnungen (7a-7c) axial und/oder radial versetzt zueinander angeordnet sind.

6. Vorrichtung nach einem der vorangehenden Ansprüche, wobei der Katheter (1, 1') in Abschnitte unterteilt ist, die jeweils die gleiche Anzahl und/oder Anordnung der Seitenöffnungen (7a-7c) aufweist.

7. Vorrichtung nach einem der vorangehenden Ansprüche, wobei der Katheter (1, 1') mindestens einen Nebenkanal (1e) aufweist, der radial versetzt zu einem Hauptkanal (1d) angeordnet ist und der den Ballon (2) fluidisch mit einem Anschluss (5) verbindet.

8. Vorrichtung nach einem der vorangehenden Ansprüche, wobei das proximale Ende (1a, 1a') des Katheters (1, 1') eine Endöffnung (6) aufweist.

9. Vorrichtung nach einem der vorangehenden Ansprüche, wobei der Katheter (1') am proximalen Ende (1a') ein Einwegventil (11) aufweist.

10. Vorrichtung nach einem der vorangehenden Ansprüche, wobei der Katheter ein Aussenkatheter (1, 1') ist, in welchen zumindest ein Teil eines Innenkatheters (20, 20') aufnehmbar ist.

11. Vorrichtung nach Anspruch 10, wobei der Innenkatheter (20, 20') einen Anschluss (24) zum Einleiten von Sklerosierungsmittel und mindestens eine Öffnung (27, 27') aufweist, durch welche hindurch Sklerosierungsmittel zu den Seitenöffnungen (7a-7c) des Aussenkatheters (1, 1') leitbar ist, wenn der Innenkatheter (20, 20') im Aussenkatheter (1, 1') eingeführt ist.

12. Vorrichtung nach einem der Ansprüche 10-11, wobei der Innenkatheter (20) ein verschlossenes proximales Ende (20a) und anschliessend dazu einen Teilabschnitt mit mindestens einer Seitenöffnung (27) aufweist.

13. Vorrichtung nach Anspruch 12, wobei anschliessend zum Teilabschnitt der Innenkatheter (20) bis zum distalen Ende (20b) verschlossen ausgebildet ist.

14. Vorrichtung nach einem der Ansprüche 10-13, wobei der Innenkatheter (20) Seitenöffnungen (27) mit einer Anzahl aufweist, die kleiner als die Anzahl der Seitenöffnungen (7a-7c) des Aussenkatheters (1) ist, vorzugsweise stimmt die Anzahl und besonders bevorzugt auch die Anordnung der Seitenöffnungen (27) des Innenkatheters (20) mit auf einem Abschnitt des Aussenkatheters (1) angeordneten Seitenöffnungen (7a-7c) überein.

15. Vorrichtung nach einem der Ansprüche 10-14, wobei der Innenkatheter (20') am proximalen Ende (26') eine Endöffnung aufweist (27') und vorzugsweise vom proximalen bis zum distalen Ende verschlossen ausgebildet ist.

16. Vorrichtung nach einem der vorangehenden Ansprüche, wobei der Aussenkatheter (1, 1') und/oder Innenkatheter (20, 20') Markierungen (8, 28) zur Längenangabe aufweist, die vorzugsweise in regelmässigen Abständen angeordnet sind.

## Claims

1. Device for the treatment of varicose veins, comprising a catheter (1, 1'), which is insertable into a vein to be treated, wherein the catheter comprises side openings (7a-7c), through which a sclerosing agent can be introduced into the vein, **characterized in that** a balloon (2) with at least one cutting element (3) for cutting at least part of the inner wall of the vein is arranged on the proximal end (1a, 1a') of the catheter.

2. Device according to claim 1, wherein the catheter (1, 1') extends in the axial direction (A) and wherein the at least one cutting element (3) comprises a cutting edge (3c), which, at least in the inflated state of the balloon (2), has a shape, which changes in an axial and/or radial direction.

3. Device according to any of the preceding claims, wherein the cutting element (3) is curved in at least one section and/or has a height, which decreases when looking towards the distal end (1b).

4. Device according to any of the preceding claims, wherein the ends (3a, 3b) of the cutting element (3) are arranged radially offset at an angle which is greater than 0 degrees and/or smaller than 360 degrees when viewed in the axial direction (A) in which the catheter (1, 1') extends, preferably the angle is at least 10 degrees and/or at most 180 degrees, most preferably the angle is at least 20 degrees and/or at most 90 degrees.

5. Device according to any of the preceding claims, wherein two adjacent side openings (7a-7c) are arranged axially and/or radially offset from one another.

6. Device according to any of the preceding claims, wherein the catheter (1, 1') is divided into sections, which each have the same number and/or arrangement of side openings (7a-7c).

7. Device according to any of the preceding claims, wherein the catheter (1, 1') comprises at least one subchannel (1e), which is arranged radially offset from a main channel (1d) and which provides a fluid connection between the balloon (2) and an access (5).

8. Device according to any of the preceding claims, wherein the proximal end (1a, 1a') of the catheter (1, 1') comprises an end opening (6).

9. Device according to any of the preceding claims, wherein the catheter (1') comprises a one-way valve (11) at the proximal end (1a').

10. Device according to any of the preceding claims, wherein the catheter is an outer catheter (1, 1'), in which at least a part of an inner catheter (20, 20') can be received.

11. Device according to claim 10, wherein the inner catheter (20, 20') comprises an access (24) for the introduction of sclerosing agent and at least one opening (27, 27'), through which sclerosing agent can be conducted to the side openings (7a-7c) of the outer catheter (1, 1') when the inner catheter (20, 20') is inserted into the outer catheter (1, 1').

12. Device according to any of claims 10 to 11, wherein the inner catheter (20) comprises a closed proximal end (20a) and adjacent thereto a portion with at least one side opening (27).

13. Device according to claim 12, wherein the part of the inner catheter (20) after said portion to the distal end (20b) has a closed design.

14. Device according to any one of claims 10 to 13, wherein the inner catheter (20) comprises a number of side openings (27), which is smaller than the number of side openings (7a-7c) on the outer catheter (1), preferably the number and most preferably also the arrangement of the side openings (27) of the inner catheter (20) match with side openings (7a-7c) arranged on a section of the outer catheter (1).

15. Device according to any one of claims 10 to 14, wherein the inner catheter (20') comprises an end opening (27') on the proximal end (26') and preferably has a closed design from the proximal to the distal end.

16. Device according to any of the preceding claims, wherein the outer catheter (1, 1') and/or the inner catheter (20, 20') comprises markings (8, 28) for indicating a length, preferably the markings are arranged at regular intervals.

## Revendications

1. Dispositif de traitement des varices, comprenant un cathéter (1, 1') pouvant être introduit dans une veine à traiter, le cathéter ayant des ouvertures latérales (7a-7c) par lesquelles un agent sclérosant est introductible dans la veine, **caractérisé en ce qu'**à l'extrémité proximale (1a, 1a') du cathéter est agencé un ballon (2) muni d'au moins un élément tranchant (3) servant à sectionner au moins une partie de la paroi intérieure de la veine.

2. Dispositif selon la revendication 1, où le cathéter (1, 1') s'étend en direction axiale (A) et où ledit au moins un élément tranchant (3) comprend une arête tranchante (3c) dont la forme change, du moins à l'état gonflé du ballon (2), en direction axiale et/ou radiale.

3. Dispositif selon l'une des revendications précédentes, où l'élément tranchant (3) est incurvé au moins sur une section partielle et/ou présente une hauteur qui diminue vers l'extrémité distale (1b).

4. Dispositif selon l'une des revendications précédentes où, vues dans la direction axiale (A) dans laquelle s'étend le cathéter (1, 1'), les extrémités (3a, 3b) de l'élément tranchant (3) sont agencées de manière radialement décalée à un angle qui est supérieur à 0 degrés et/ou inférieur à 360 degrés, l'angle est préférablement égal ou supérieur à 10 degrés et/ou inférieur ou égal à 180 degrés, l'angle est particulièrement préférablement égal ou supérieur à 20 degrés et/ou inférieur ou égal à 90 degrés.

5. Dispositif selon l'une des revendications précédentes, où deux ouvertures latérales (7a-7c) adjacentes sont agencées de manière axialement et/ou radialement décalée l'une par rapport à l'autre.

6. Dispositif selon l'une des revendications précédentes, où le cathéter (1, 1') est divisé en sections qui présentent chaque fois le même nombre et/ou le même agencement des ouvertures latérales (7a-7c).

7. Dispositif selon l'une des revendications précédentes, où le cathéter (1, 1') comprend au moins un canal secondaire (1e) agencé de manière radialement décalée par rapport à un canal principal (1d) et qui relie fluidiquement le ballon (2) à un raccordement (5).

8. Dispositif selon l'une des revendications précédentes, où l'extrémité proximale (1a, 1a') du cathéter (1, 1') présente une ouverture d'extrémité (6).

9. Dispositif selon l'une des revendications précédentes, où le cathéter (1') comprend à son extrémité proximale (1a') une soupape de non-retour (11).

10. Dispositif selon l'une des revendications précédentes, où le cathéter est un cathéter extérieur (1, 1') pouvant recevoir au moins une partie d'un cathéter intérieur (20, 20').

11. Dispositif selon la revendication 10, où le cathéter intérieur (20, 20') comprend un raccordement (24) pour l'introduction d'un agent sclérosant et au moins une ouverture (27, 27') par laquelle l'agent sclérosant peut être amené aux ouvertures latérales (7a-7c) du cathéter extérieur (1, 1') lorsque le cathéter intérieur (20, 20') est introduit dans le cathéter extérieur (1, 1').

12. Dispositif selon l'une des revendications 10-11, où le cathéter intérieur (20) comprend une extrémité proximale (20a) fermée qui est prolongée par une section munie d'au moins une ouverture latérale (27).

13. Dispositif selon la revendication 12, où au-delà de ladite section, le cathéter intérieur (20) est fermé jusqu'à l'extrémité distale (20b).

14. Dispositif selon l'une des revendications 10-13, où le cathéter intérieur (20) présente des ouvertures latérales (27) d'un nombre qui est inférieur au nombre d'ouvertures latérales (7a-7c) du cathéter extérieur (1), le nombre et particulièrement également l'agencement des ouvertures latérales (27) du cathéter intérieur (20) correspond préférablement aux ouvertures latérales agencées (7a-7c) sur une section du cathéter extérieur (1).

15. Dispositif selon l'une des revendications 10-14, où le cathéter intérieur (20') présente à son extrémité proximale (26') une ouverture d'extrémité (27') et est préférablement fermé de l'extrémité proximale jusqu'à l'extrémité distale.

16. Dispositif selon l'une des revendications précédentes, où le cathéter extérieur (1, 1') et/ou le cathéter intérieur (20, 20') présentent des marquages (8, 28) d'indication de la longueur qui sont préférablement pourvus à des intervalles réguliers.
